# EUROPÄISCHE PATENTSCHRIFT

(11) **EP 0 891 963 B1**
(45) Veröffentlichungstag und Bekanntmachung des Hinweises auf die Patenterteilung: **16.10.2002**
(21) Anmeldenummer: 98112816.8
(22) Anmeldetag: 10.07.1998
(51) Int. Cl.: C07C 51/09, C07C 53/126, C07C 57/03

(54) **Verfahren zur Herstellung von Fettsäuren**
Process for the preparation of fatty acids
Procédé de préparation d'acides gras

(30) Priorität: 18.07.1997 DE 19730863
(43) Veröffentlichungstag der Anmeldung: 20.01.1999
(73) Patentinhaber: Cognis Deutschland GmbH & Co. KG, 40589 Düsseldorf (DE)
(72) Erfinder: Gritz, Egbert Dr., 40597 Düsseldorf (DE); Gutsche, Bernhard Dr., 40724 Hilden (DE); Lock, Thorsten, 40589 Düsseldorf (DE); Steinberner, Udo Dr., 40724 Hilden (DE); Wollmann, Gerhard Dr., 40723 Hilden (DE)

(56) Entgegenhaltungen:
- EP-A- 0 596 484
- DE-A- 2 710 630
- FR-A- 1 602 512
- US-A- 4 185 027

## Beschreibung

### Gebiet der Erfindung

Die Erfindung befindet sich auf dem Gebiet der Oleochemie und betrifft ein Verfahren zur Herstellung von Fettsäuren durch Hydrolyse von Fettsäurealkylestern in Gegenwart eines speziellen heterogenen sauren Katalysators.

### Stand der Technik

Aus dem Stand der Technik sind verschiedene Verfahren zur Gewinnung von Fettsäuren durch Umsetzung von Fettsäurealkylestern, insbesondere Fettsäuremethylestem bekannt. Es handelt sich dabei vorzugsweise um Verfahren zur Gewinnung der kurzkettigen sogenannten Vorlauf-Fettsäuren.

Ein Verfahren zur Gewinnung von Fettsäuren ist die Verseifung des entsprechenden Methylesters und anschließende Seifenspaltung. Üblicherweise erfolgte die Gewinnung der Fettsäure in einem zweistufigen Verfahren, bei dem zunächst der Fettsäurealkylester mit Glycerin zum Triglycerid umgesetzt wird und dieses dann anschließend in Fettsäure und Glycerin gespalten wird. Die sogenannte Fettspaltung kann unter verschiedenen Bedingungen durchgeführt werden. Gemäß dem Twitchell-Verfahren arbeitet man kontinuierlich oder diskontinuierlich unter Atmosphärendruck in Gegenwart von Schwefelsäure oder Alkylarylsulfonsäuren. Daneben ist auch als kontinuierlicher Prozess das Hoffmann-Verfahren bekannt, bei dem die Umsetzung im Rohrreaktor bei 25 bis 50 bar und 220 bis 260 °C im Gegenstrom erfolgt. Auch Enzyme lassen sich zur Fettspaltung einsetzen, doch großtechnisch wird am häufigsten die Umsetzung mit Wasser unter Druck genutzt. Aus der **DE 3403021 A1** ist ein Verfahren zur Gewinnung von Fettsäuren durch einen zweistufigen Prozess bekannt.

Neben den zweistufigen Verfahren sind jedoch auch solche bekannt, bei denen die Fettsäurealkylester direkt gespalten werden. Eine derartige Spaltung kann sowohl durch Säuren als auch durch Basen herbeigeführt werden. Um einen möglichst hohen Umsatz zu erreichen, müssen beide in hohem Überschuss eingesetzt werden. Anschließend wird das Produkt üblicherweise gewaschen, getrocknet und destilliert. Nachteile der Verfahren ergeben sich einerseits durch die hohen Kosten für die Prozesschemikalien, eine Verlangsamung der Reaktion mit zunehmender Kettenlänge des eingesetzten Alkylesters und durch die Bildung von Nebenprodukten wie beispielsweise Seifen.

Die Säurespaltung von Fettsäurealkylestern erfolgt i.a. mittels kurzkettiger Carbonsäuren, vorzugsweise Propionsäure. Ein derartiger Prozess wird beispielsweise in der Patentschrift **US 1,882,808** beschrieben. Grundsätzlich sind auch zur Spaltung der Fettsäurealkylester die bereits von der Spaltung der Triglyceride bekannten Verfahren, wie beispielsweise die Spaltung mit Wasser unter erhöhtem Druck oder eine enzymatische Spaltung möglich. Doch ergeben sich bei den Verfahren unter hohem Druck aufgrund der technischen Anforderungen recht hohe Kosten, während die enzymatische Spaltung häufig nicht vollständig verläuft und die Auswahl der Enzyme sowie die Prozesskontrolle kritisch sind. Aus der **WO 94/14743** ist ein Verfahren zur Herstellung von Fettsäuren durch sauer katalysierte Hydrolyse von Fettsäuremethylestem bekannt. Als homogene Katalysatoren werden vorzugsweise Alkylbenzolsulfonsäuren eingesetzt. Es handelt sich dabei um oberflächenaktive Substanzen, die eine bessere Durchmischung der wässrigen Phase mit dem Ester bewirken sollen. Durch spezielle Mischungsverhältnisse von Ester, Wasser und Katalysator soll erreicht werden, dass die Reaktion in homogener Phase abläuft. Die Reaktion wird bei Temperaturen im Bereich von 70 bis 110 °C durchgeführt Bei dieser Methode ergibt sich jedoch ein erheblicher Nachteil durch hohe Katalysatorverluste. Gleichzeitig erhält man eine Fettsäure von minderer Qualität, da sie noch Nebenprodukte enthält

Aus der **US 4,185,027** ist ein Verfahren zur Hydrolyse von Methylestern unter Einsatz von sauren Katalysatoren bekannt, bei denen es sich u.a. auch um saure Kationenaustauscher beispielsweise vom Amberlite- bzw. Amberlyst-Typ handeln kann. Die **DE-OS 2710630** behandelt ein ähnliches Verfahren, bei dem ein fester saurer Si-Ti-Katalysator zum Einsatz gelangt. Auch aus der **FR-B1 1602512** ist der Einsatz von sauren Ionenaustauschern für die Spaltung von Estern bekannt. Schließlich wird in der **EP 0596484 A1** über die Herstellung von Ameisensäure unter Einsatz von Sulfonsäure-Kationenaustauschern berichtet.

Die komplexe Aufgabe der vorliegenden Erfindung war es, ein Verfahren zur Herstellung von Fettsäuren durch Spaltung von Fettsäurealkylestern zu entwickeln, welches technisch leicht durchzuführen ist, Katalysatorverluste vermeidet und gleichzeitig die direkte Umsetzung des Alkylesters zur Fettsäure ermöglicht. Weiterhin sollten Fettsäuren hoher Qualität erhalten werden, ohne dass aufwendige Raffinationsverfahren eingesetzt werden müssen.

### Beschreibung der Erfindung

Gegenstand der Erfindung ist ein Verfahren zur Herstellung von Fettsäuren durch Hydrolyse von Fettsäurealkylestern der Formel **(I),**

**R**^{**1**}**COOCH**_{**3**} **(I)**

in der R¹CO für einen aliphatischen, linearen oder verzweigten Acylrest mit 6 bis 12 Kohlenstoffatomen steht, in Gegenwart von heterogenen sauren Katalysatoren, welches sich dadurch auszeichnet, dass man als Katalysatoren sulfonsäurefixierte Kieselgele einsetzt.

Es wurde nun überraschenderweise gefunden, dass durch Einsatz des speziellen heterogenen sauren Katalysators die aus der Technik bekannten Nachteile vermieden werden können. Man erreicht eine Umsetzung des Alkylesters direkt in einer Stufe zur korrespondierenden Fettsäure ohne dabei hohe Katalysatorverluste oder den Verbrauch teurer Prozesschemikalien in Kauf zu nehmen.

### Fettsäurealkylester

Als Ausgangsprodukte eignen sich vorzugsweise C₁ bis C₄- Alkylester pflanzlicher und/ oder tierischer Fettsäuren. Typische Beispiele sind Capronsäure, Caprylsäure, 2-Ethylhexansäure, Caprinsäure, Laurinsäure, Isotridecansäure, Myristinsäure, Palmitinsäure, Palmoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen. Im Sinne der vorliegenden Erfindung sind insbesondere Fettsäuremethylester, vorzugsweise mit 6 bis 12 C-Atomen im Acylrest, als Ausgangsprodukte geeignet, besonders bevorzugt sind die sogenannten Vorlauffettsäuremethylester. Zur Herstellung von Fettsäuremethylestern - welche als Ausgangsmaterialien beispielsweise für Fettalkohole und deren Folgeprodukte eine hohe Bedeutung besitzen - bedient man sich üblicherweise der Umesterung natürlich vorkommender Fette und Öle mit niederen Alkoholen, üblicherweise C1 bis C4. Dabei erhält man Estergemische, deren Fettsäureanteile aus C₁₂ bis C₁₈-Fettsäuren bestehen. Bei einigen Ausgangsmaterialien, insbesondere bei Kokosund Palmkernöl erhält man daneben jedoch noch eine Fraktion mit Estern kurzkettiger Fettsäuren, die im allgemeinen durch Destillation abgetrennt werden. Es handelt sich dabei im allgemeinen um Ester der C₆ bis C₁₀ Fettsäuren, die sogenannten Vorlauffettsäuren. Im Sinne der vorliegenden Erfindung werden bevorzugt diese im Rahmen der Umesterung von Palmkern- und Kokosöl anfallenden Vorlauffettsäureester als Ausgangsprodukte für die Gewinnung von Fettsäuren eingesetzt. Der Säureanteil dieser Ester besteht üblicherweise aus Capryl-, Capron- und Caprinsäure. In untergeordneten Mengen kann beispielsweise auch Laurinsäure anwesend sein. Die Mengenverhältnisse in denen die unterschiedlichen Fettsäuren in den Vorlauffettsäureestern vorkommen sind in Abhängigkeit von der Herkunft der nativen Rohstoffe und der Durchführung der destillativen Trennung weiten Schwankungen unterworfen. Als typische Beispiele für die Zusammensetzung des Säureanteils von erfindungsgemäß als Ausgangsmaterial zu verwendeten Vorlauffettsäureestern seien die folgenden genannt:

### Vorlauffettsäureschnitt aus Kokosöl

| | |
|---|---|
| Capronsäure | Spuren |
| Caprylsäure | 88,5 Gew.-% |
| Caprinsäure | 11,5 Gew.-% |

### Vorlauffettsäureester aus Palmkernöl

| | |
|---|---|
| Capronsäure | 1,2 Gew.-% |
| Caprylsäure | 59,7 Gew.-% |
| Caprinsäure | 34,6 Gew.-% |
| Laurinsäure | 4,5 Gew.-% |

Als Alkoholkomponente der Vorlauffettsäurealkylester kommen Ethanol, n-Propanol, Isopropylalkohol, n-Butanol, sek.-Butanol, tert.-Butanol und vorzugsweise Methanol in Betracht.

### Katalysator

Bei den Katalysatoren handelt es sich um Kieselgele deren saure Funktionalität durch Sulfonsäuren bedingt ist, sogenannte sulfonsäurefixierte Kieselgele, insbesondere um thermound druckstabile sogenannte sulfonsäurefixierte Kieselgele. Diese erhält man beispielsweise wie in der **Dissertation von U. Kalin, Universität Duisburg 1990** beschrieben, hierbei wird Kieselgel mit α-Chlorpropyl-trichlorsilan behandelt anschließend hydrolysiert und sulfoniert. Im Rahmen der vorliegenden Erfindung einzusetzende Katalysatoren werden auch von der Firma Degussa unter dem Handelsnamen Deloxan ASP® vertrieben. **(CLB Chemie in Labor und Biotechnik, 43 (1992) 1, S. 16 bis 21).** Es handelt sich erfindungsgemäß um heterogene Katalysatoren, die vorzugsweise als Festbettkatalysatoren eingesetzt werden. Die Teilchengröße beträgt dabei bevorzugt 0,2 bis 5 mm, insbesondere 1 bis 3 mm. Bevorzugt werden Katalysatoren mit einer Ionenaustauscherkapazität von 0,5 bis 3 mMol/g, insbesondere 0,9 bis 1,2 mMol/g.

### Verfahren

Die Reaktion wird vorzugsweise in einem zylindrischen senkrecht stehenden Reaktor mit Katalysatorfestbett in einem oder mehreren Segmenten durchgeführt. Am Reaktorkopf speist man gegebenenfalls über eine Vorheizstrecke, die flüssige Fettphase ein, die durch das Katalysatorbett zum Kolonnenboden rieselt, im Gegenstrom wird von dort der Wasserdampf - gegebenenfalls auch über eine beheizte Gasleitung - eingespeist. Vorzugsweise erfolgt die Einspeisung der Fettphase unterhalb des Dampfabzuges und oberhalb der Fetteinspeisung wird zusätzlich ein Rektifikationskolonnenaufsatz angebracht. Die Reaktion wird üblicherweise bei einer Temperatur im Bereich von 130 bis 250, vorzugsweise 160 bis 180 °C und unter erhöhtem Druck von 2 bis 15, vorzugsweise 4 bis 10 bar durchgeführt. Grundsätzlich kann die Reaktion auch unter Atmosphärendruck durchgeführt werden, was jedoch zu längeren Reaktionszeiten und niedrigeren Ausbeuten führt.

In einer weiteren Ausführungsform wird das Wasser gemeinsam mit der Fettphase am Reaktorkopf eingespeist, während im Gegenstrom vom Reaktorboden ein Inertgas durch den Reaktor geleitet wird, bevorzugt handelt es sich um Stickstoff. In diesem Fall liegt die Reaktortemperatur unterhalb des Siedepunktes von Wasser, die Reaktion kann bei Atmosphärendruck oder auch unter erhöhtem Druck durchgeführt werden. Am Austritt der Gasphase - am Kolonnenkopf - befindet sich vorzugsweise ein gekühlter Flüssigkeitsabscheider, in dem sich die durch das Gas mitgerissenen Anteile an Fettsäure, Alkylester und Wasser sammeln. Nach Phasentrennung kann die Fettphase erneut in die Reaktion eingespeist werden.

Der Produktaustritt am Reaktorboden erfolgt üblicherweise über einen Gasabscheider in einen Vorlagebehälter.

### Beispiele

Die Versuche wurden in einem 1I-Druckreaktor mit einem Durchmesser von 6,2 cm und einer Füllhöhe von 35 cm durchgeführt. Die Fettphase wurde am Reaktorkopf über eine Vorheizstrecke flüssig bei einer Temperatur von ca. 170 °C eingeführt und rieselte durch das Katalysatorbett zum Kolonnenboden. Unterhalb des Katalysatorfestbettes befand sich eine beheizte Gaszuleitung (T = 15 bis 180 °C). In den Versuchen V1 bis V7 wurde über die untere Zuleitung Wasserdampf eingespeist; während bei den Versuchen V10 bis V16 über die untere Zuleitung Stickstoff eingespeist wurde und Wasser in flüssiger Form gemeinsam mit der Fettphase am Reaktorkopf zugeführt wurde. Der Gasausschluss am Reaktorkopf war mit einem Flüssigkeitsabscheider, Kühler und Druckhalterventil ausgestattet, der Produktaustritt am Reaktorboden erfolgte über einen Gasabscheider in einen Vorlagenbehälter. Die Flüssigkeiten wurden mit Labordosierpumpen gefördert. Die Reaktortemperatur betrug 170 °C, die Versuche wurden unter erhöhtem Druck mit Deloxan® 50 als Festbettkatalysator durchgeführt. Der stückige Katalysator, mit einer Körnung von 1 bis 3 mm war in einem senkrechten zylindrischem Reaktor angeordnet. Als Edukt wurde Vorlauffettsäuremethylester C₈/C₁₀ (1:1) eingesetzt. Die Ergebnisse sind in Tabelle 1 dargestellt.

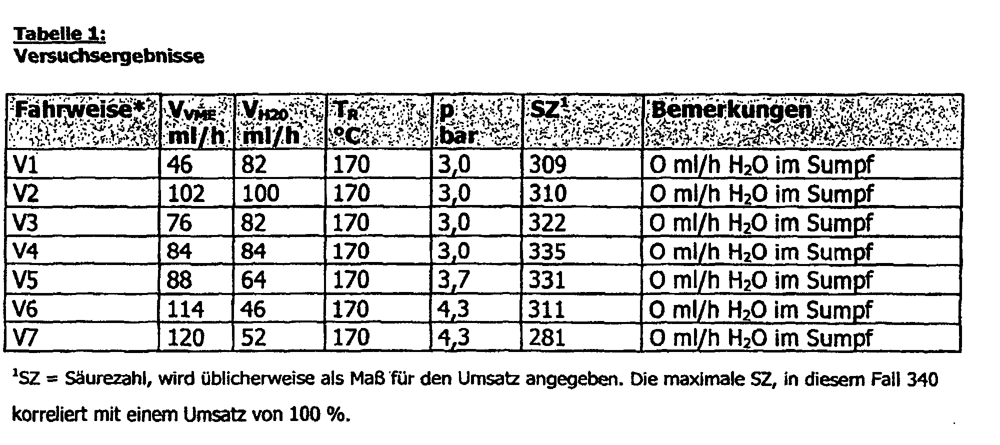

## Patentansprüche

1. Verfahren zur Herstellung von Fettsäuren durch Hydrolyse von Fettsäurealkylestern der Formel **(I)**,
**R**^{**1**}**COOCH**_{**3**} **(I)**
in der R¹CO für einen aliphatischen, linearen oder verzweigten Acylrest mit 6 bis 12 Kohlenstoffatomen steht, in Gegenwart von heterogenen sauren Katalysatoren, **dadurch gekennzeichnet, dass** man als Katalysatoren sulfonsäurefixierte Kieselgele einsetzt.

2. Verfahren nach Anspruch 1, **dadurch gekennzeichnet, dass** man C₆-C₁₀-Fettsäuremethylester einsetzt, die bei der Fraktionierung der durch Umesterung von Palmkern- oder Kokosöl gewonnenen Fettsäurealkylester als leichtsiedende Fraktion, anfallen.

3. Verfahren den Ansprüchen 1 und/oder 2, **dadurch gekennzeichnet, dass** man die Katalysatoren im Festbett einsetzt.

4. Verfahren nach mindestens einem der Ansprüche 1 bis 3, **dadurch gekennzeichnet, dass** man die Reaktion im Gegenstromverfahren durchführt.

## Claims

1. A process for the production of fatty acids by hydrolysis of fatty acid alkyl esters corresponding to formula (I):
**R**^{**1**}**COOCH**_{**3**} **(I)**
in which R¹CO is an aliphatic, linear or branched acyl group containing 6 to 12 carbon atoms,
in the presence of heterogeneous acidic catalysts, **characterized in that** sulfonic-acid-fixed silica gels are used as catalysts.

2. A process as claimed in claim 1, **characterized in that** C₆₋₁₀ fatty acid methyl esters accumulating as low-boiling fraction in the fractionation of fatty acid alkyl esters obtained by transesterification of palm kernel oil or coconut oil are used.

3. A process as claimed in claims 1 and/or 2, **characterized in that** the catalysts are used in a fixed bed.

4. A process as claimed in at least one of claims 1 to 3, **characterized in that** the reaction is carried out on the countercurrent principle.

## Revendications

1. Procédé de préparation d'acides gras par hydrolyse d'esters d'acide gras de formule (I)
R¹COOCH₃ (I)
dans laquelle R¹CO représente un reste acyle aliphatique, linéaire ou ramifié ayant de 6 à 12 atomes de carbone, en présence de catalyseurs hétérogènes, acides,
**caractérisé en ce qu'**
on met en oeuvre comme catalyseurs des gels de silice fixés par des acides sulfoniques.

2. Procédé selon la revendication 1,
**caractérisé en ce qu'**
on met en oeuvre des esters méthyliques d'acide gras en C₆-C₁₀ qui se forment lors du fractionnement des esters d'alkyle produits par transestérification d'huile de palmiste ou de coco, en tant que fraction à bas point d'ébullition.

3. Procédé selon l'une quelconque des revendications 1 et/ou 2,
**caractérisé en ce qu'**
on met en oeuvre les catalyseurs en lit fixe.

4. Procédé selon au moins l'une quelconque des revendications 1 à 3,
**caractérisé en ce qu'**
on effectue la réaction en un procédé à contre-courant.
